# EUROPÄISCHE PATENTANMELDUNG

(11) **EP 1 262 810 A2**
(43) Veröffentlichungstag der Anmeldung: **04.12.2002**
(21) Anmeldenummer: 02011904.6
(22) Anmeldetag: 29.05.2002
(51) Int. Cl.: G02B 21/00

(54) **Verfahren zur Entfernungsmessung ausgedehnter Objekte in Verbindung mit einer optischen Betrachtungseinrichtung und Mikroskop zur Durchführung desselben**

(30) Priorität: 29.05.2001 DE 10125971
(71) Anmelder: Leica Microsystems AG, 9435 Heerbrugg (CH)
(72) Erfinder: Spink, Roger, 9442 Berneck (CH)
(74) Vertreter: Reichert, Werner F., Dr.

(57) **Zusammenfassung**

Verfahren und Vorrichtung zur Bestimmung eines oder mehrerer Z-Abstände von einer Objekt-Oberfläche (12) zu einer Bezugsebene, beispielsweise der Hauptebene des Hauptobjektivs (10) eines Mikroskops. Durch die Projektion eines optischen Musters (51) auf ein im Bereich einer Fokus-Ebene (13) befindliches Objekt (12), anschließender Detektion und rechnerischer Auswertung der Objekt-Reflexion dieses Musters mittels einer Bildverarbeitungseinheit (61) wird, unabhängig von der Objektkonturierung, eine reliefartige Abbildung des Objektes (12) und die Ermittlung der einzelnen z-Abstände möglich.

## Beschreibung

Die Erfindung betrifft ein neuartiges Verfahren zur Bestimmung eines oder mehrerer Z-Abstände von einer Oberfläche zu einer Bezugsebene, beispielsweise der Hauptebene des Hauptobjektivs einer optischen Betrachtungseinrichtung, beispielsweise eines (Stereo-) Operations-Mikroskops, das eine räumliche Abbildung des Objektes liefert, sowie ein Mikroskop zur Durchführung eines derartigen Verfahrens.

Die Bestimmung des Abstandes vom Operationsmikroskop zum Objekt ist insbesondere in der Chirurgie - beispielsweise bei neurochirurgischen Anwendungen - von besonderem Interesse, da häufig mit Navigationssystemen operiert und das Mikroskop als berührungsfreier Sensor eingesetzt wird. Wird über das definierte Messintegral erfolgreich fokussiert, beispielsweise über einer begrenzten Fläche um den Fokuspunkt, kann der Messwert an das Navigationssystem übergeben, dort verarbeitet und anschließend im Datensatz des Objektes, in der Regel des Patienten, indiziert werden.

Bei den heute bekannten Fokussystemen wird zur Bestimmung des Arbeitsabstandes eines Stereo-Mikroskops zur Objekt-Oberfläche der nach der Optimierung der Bildschärfe eingestellte Arbeitsabstand herangezogen. Die Bewertung der Bildschärfe kann im gesamten oder einem abgegrenzten Gesichtsfeldbereich erfolgen, beispielsweise in einem eng umrissenen Bereich des Gesichtsfeldzentrums, üblicherweise fast punktförmig mit ca. 1 bis 2 mm Durchmesser. Die Fokussierung erfolgt in der Regel über das Hauptobjektiv.

Der Erfinder erkannte, dass die bekannten Systeme, in Bezug auf die folgenden Punkte nachteilig sind:
a) Verfahren dieser Art benötigen im allgemeinen eine Mindest-Objektkonturierung (Randschärfe an den Kanten) mit entsprechendem Kontrast. Ist diese Voraussetzung nicht gegeben, versagen diese Verfahren normalerweise.
b) Insbesondere anatomische Präparate, beispielsweise Gehirngewebe, weisen unter Umständen weder gut zeichnende Konturen noch eine geeignete Textur mit einem Mindest-Kontrast auf,
c) Die vielfach knapp ausreichenden Bedingungen können durch das Objekt benetzende Flüssigkeiten, beispielsweise Spülflüssigkeiten vermischt mit Blut, ungenügend sein.
d) Eine Ermittlung der Z-Abstände über das gesamte Gesichtsfeld oder über größere Bereiche des Objekts, d.h. eine reliefartige Erfassung bzw.
   Abbildung des Objektes, ist nicht möglich.
e) Die notwendige Fokussierung über das Hauptobjektiv kann den Anwender auf Grund der Veränderungen der Bildschärfe den Anwender bei seiner Arbeit stören.
f) Eine unterschiedliche Fokussierung für einen weiteren Betrachter und/oder ein Video-System ist nicht möglich, d.h. es kann kein zweiter Beobachter mit einer anderen Fokus-Ebene eingesetzt werden.

Der Erfindung liegt somit die Aufgabe zugrunde, ein Verfahren zu schaffen, welches eine genaue Entfernungsbestimmung: Objekt - Operationsmikroskop unter ungünstigen Bedingungen und/oder über das gesamte Bildfeld erlaubt sowie die angegebenen Nachteile bekannter Lösungen vermeidet.

Gelöst wird diese Aufgabe durch ein Verfahren, welches mittels eines Muster-Generators ein optisches Muster erzeugt, dieses Muster über eine Einspiegelung in den Hauptstrahlengang des Mikroskops auf das Objekt projiziert, die Reflexion auf dem Objekt über den Hauptstrahlengang wieder ausspiegelt, und mittels einer Betrachtungseinrichtung, beispielsweise einer Kamera / CCD, detektiert, diese Detektion rechnerisch in konjugierte Muster aufteilt und aus der topografischen Lage dieser konjugierten Muster den Abstand Objektrelief - Mikroskop ermittelt, sowie durch ein Mikroskop zur Durchführung des genannten Verfahrens.

Es stützt sich somit auf folgenden Effekt: Bei genauer Fokussierung des Musters auf die Fokus-Ebene erscheint das Muster als ein einziges Muster; liegt eine Defokussierung vor, erscheinen zwei konjugierte Muster.

Eine besondere Weiterentwicklung, die auch unabhängig vom Muster-Generator einsetzbar ist, besteht darin, dass der Kamera eine eigene, von der Mikroskop-Hauptoptik unabhängige Optik vorgelagert wird und durch diese die Z-Ermittlung erfolgt.

Durch den vorgängig beschriebenen Einsatz eines Verfahrens mittels Muster-Projektion auf das Objekt und nachträglicher Detektion zur Fokus-Bestimmung und gegebenenfalls durch die Anwendung weiterer Verbesserungen, die in den abhängigen Ansprüchen angegeben sind, werden die nachstehend aufgeführten Verbesserungen erreicht:
- Durch den erfindungsgemäßen Einsatz einer eigenen Lichtquelle (optisches Muster) ist keine Mindestkonturierung des Objektes notwendig;
- Kontraststörungen während der Messung - beispielsweise durch Flüssigkeiten - fallen weg;
- eine Fokussierung auf alle - insbesondere auch auf abgeblendete oder unbeleuchtete Objektteile - ist möglich;
- die Fokus-Messung ist, da sie über eine eigene Lichtquelle verfügt, unabhängig von der Hauptbeleuchtung, insbesondere unabhängig vom verwendeten Lichtwellenbereich des Beleuchtungsstrahls;
- durch die erfindungsgemäße Verwendung von optischen Mustern ist die sichere Unterscheidung von anderen Lichterscheinungen möglich. Bei großflächigen Mustern wird darüber hinaus die Ermittlung des großflächigen Reliefs des Objektes und damit auch eine Fokussierung auf verschiedene Fokus-Ebenen möglich;
- durch den erfindungsgemäßen Einsatz einer eigenen Optik für die Kamera ist eine Veränderung der Fokussierung der Hauptoptik zur Fokus-Ermittlung nicht notwendig; deshalb wird der Anwender bei seiner Arbeit unter dem Mikroskop nicht gestört.

Im obigen Text wird zwar auf ein Operationsmikroskop Bezug genommen; die Erfindung ist jedoch nicht darauf eingeschränkt, sondern steht vielmehr auch anderen Benutzern optischer Geräte mit Entfernungs- und Fokussier-Einrichtungen offen (z.B. Projektoren, Video- und Fotokameras, etc.).

Moirée-Verfahren werden von der vorliegenden Erfindungsind nicht umfasst.

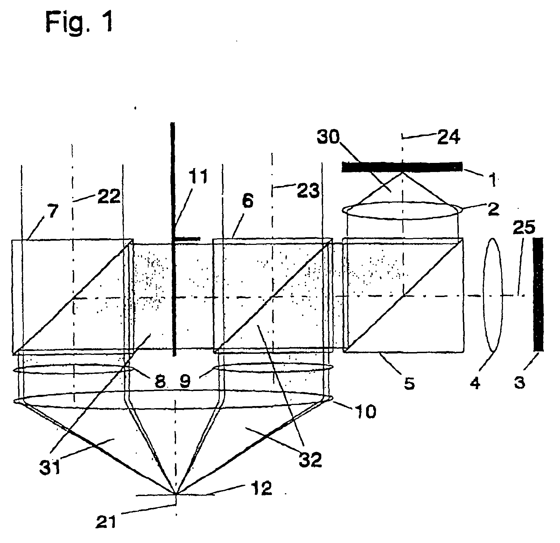

Die Bezugszeichenliste und die Fig.1 bis Fig.8 sind zusammen mit den in den Ansprüchen beschriebenen Gegenständen integrierender Bestandteil der Offenbarung dieser Anmeldung. Weitere Details und Ausführungsvarianten ergeben sich somit auch aus der Figurenbeschreibung.

### Figurenbeschreibung

Die Figuren werden zusammenhängend und übergreifend beschrieben. Gleiche Bezugszeichen bedeuten gleiche Bauteile, Bezugszeichen mit unterschiedlichen Indizes geben funktionengleiche Bauteile an. Sie zeigen symbolisch in
- Fig.1:: einen symbolischen Gesamtaufbau einer optischen Betrachtungseinrichtung, beispielsweise eines Stereo-Operations-Mikroskops;
- Fig.2:: ein Beispiel einer Blende für die (zeitliche) Kodierung der Musterelemente;
- Fig.3:: einen schematischen Verlauf eines eingespiegelten punktförmigen Musters;
- Fig.4a:: ein fokussiertes Strichmuster;
- Fig.4b:: ein konjugiertes Musterelement eines defokussierten Strichmusters;
- Fig.5:: eine Musterprojektion in verschiedenen Fokus-Ebenen;
- Fig.6:: einen Regelkreis der Fokussiereinrichtung für das Hauptobjektiv und :der Fokussiereinrichtung für die Kamera;
- Fig.7:: eine Objekt-Oberfläche und verschiedene zu detektierende Abstände z1 bis z5 zur Hauptoptik und
- Fig.8:: eine Objekt-Oberfläche und verschiedene zu detektierende Abstände z1 bis z5 zur Hauptoptik sowie eine Fokus-Ebene und zwei defokussierte Ebenen.

Die Fig.1 zeigt symbolisch den Gesamtaufbau einer optischen Betrachtungseinrichtung mit einem Muster-Generator 1, einer Optik 2 für den Muster-Generator, einer Kamera 3, beispielsweise ein CCD, der dazugehörigen Optik 4, einen Strahlenteiler 5 für den Muster-Generator 1, jeweils einen Strahlenteiler 6 und 7 für die Ein- bzw. Ausspiegelung von Mustern im linken bzw. rechten Strahlengang 31 bzw. 32 einer Zoom-Optik 8, 9 für den linken bzw. rechten Strahlengang 31 bzw. 32, einem Hauptobjektiv 10, sowie einer Blende 11 für die (zeitliche) Kodierung der Muster-Elemente. Im weiteren sind die Objekt-Oberfläche 12, die optischen Achsen, und zwar 21 für das Hauptobjektiv 10, für den linken bzw. rechten Strahlengang 22 bzw. 23, die optische Achse 24 des Muster-Generators 1, sowie die optische Achse 25 mit dem Strahlengang der Kamera 3 abgebildet.

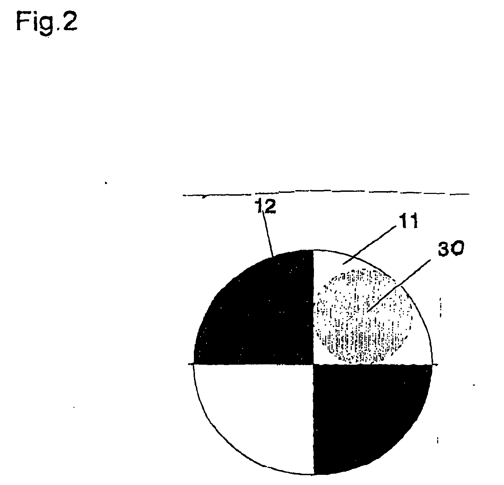

Fig.2 zeigt schematisch eine Blende 11, beispielsweise eine rotierende Scheibe, mit der Objekt-Oberfläche 12 und dem eingespiegelten Strahlengang 30 des Muster-Generators 1 für die (zeitliche) Kodierung der rechten bzw. linken Perspektive des Musters zwecks Auswertung über eine Kamera 3.

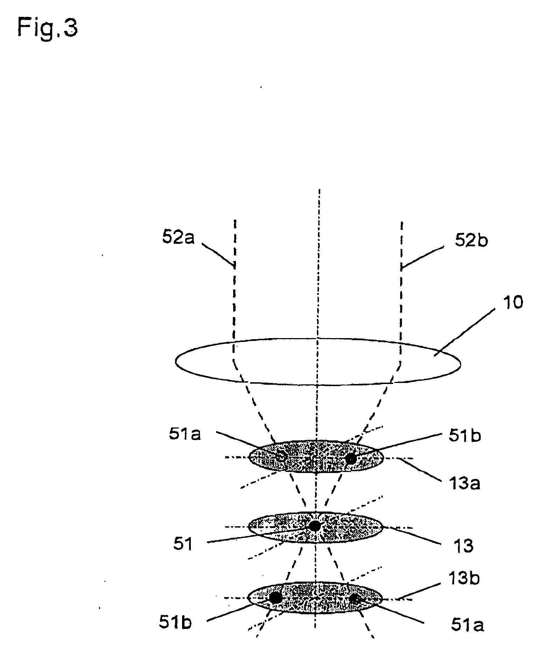

Fig.3 zeigt den Verlauf eines eingespiegelten punktförmigen Musters 52a, 52b, das Hauptobjektiv 10, das fokussierte Muster 51, die defokussierten Musterelemente 51a, 51b sowie die Fokus-Ebene 13, beispielsweise die Objekt-Oberfläche, und die defokussierten Ebenen 13a, 13b.

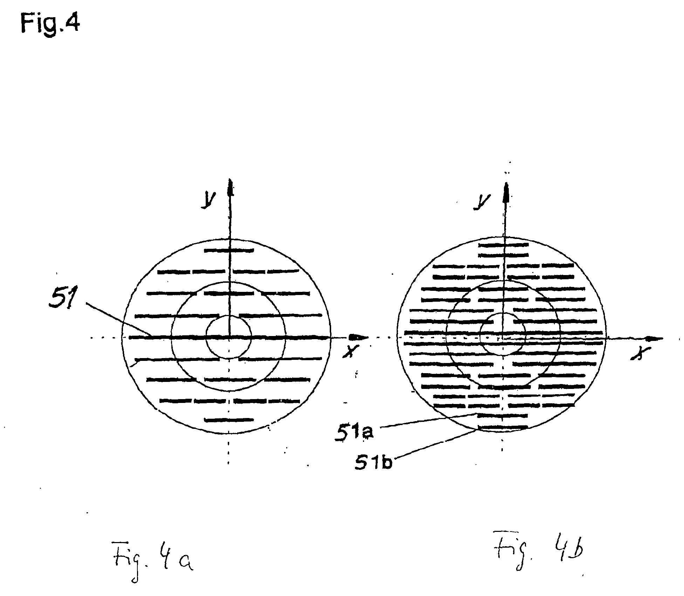

Fig.4a zeigt symbolisch ein fokussiertes Strichmuster 51; die Musterelemente 51a und 51b liegen präzise übereinander. Fig. 4b zeigt die konjugierten Musterelemente 51a, 51b eines defokussierten Strichmusters.

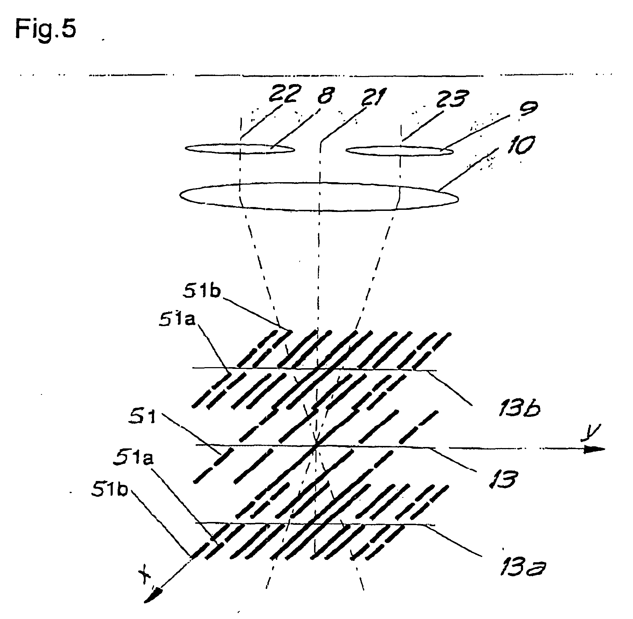

In Fig.5 wird das in Fig.4 symbolisch gezeichnete Strichmuster schematisch dargestellt mit der Fokus-Ebene 13, den defokussierten Ebenen 13a, 13b sowie dem fokussierten Muster 51 bzw. defokussierten Mustern 51a, 51b.

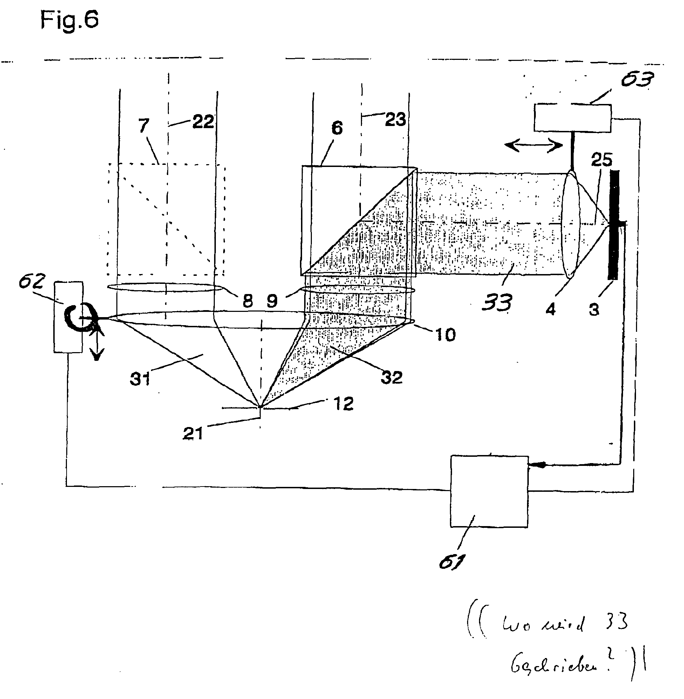

Fig.6 zeigt schematisch den Regelkreis, bestehend aus Kamera 3, Bildverarbeitungseinheit 61 zur Berechnung des effektiven Fokuspunktes aus den aktuellen Fokusparametern von Hauptobjektiv 10 und Kamara-Objektiv 4, einer Fokussiereinrichtung 62 für das Hauptobjektiv 10 sowie einer Fokussiereinrichtung 63 für die Optik 4 der Kamera 3.

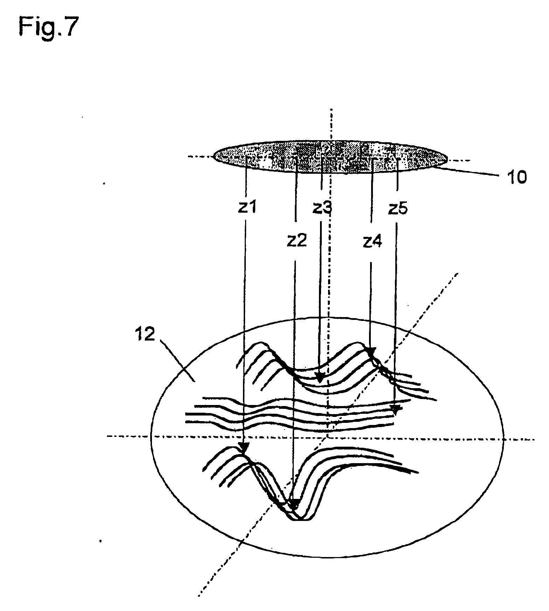

Fig.7 zeigt symbolisch eine Objekt-Oberfläche 12 und verschiedene zu detektierende Abstände z1 bis z5 zur Hauptoptik 10.

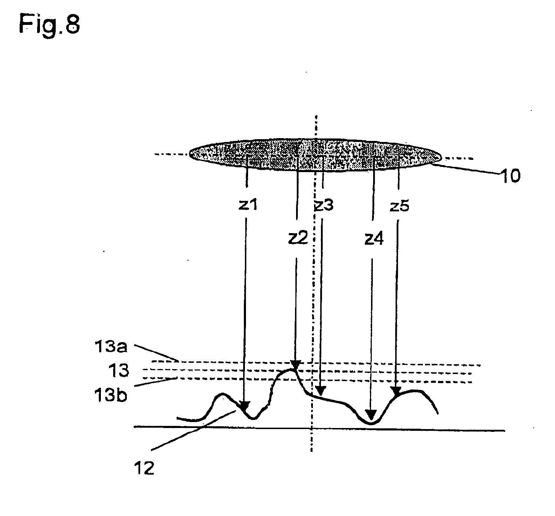

Fig.8 zeigt analog zu Fig.7 eine Objekt-Oberfläche 12, verschiedene zu detektierende Abstände z1 bis z5 zur Hauptoptik 10 sowie die Fokus-Ebene 13, beispielsweise zu z2, sowie die defokussierten Ebenen 13a, 13b.

### Funktionsweise

In einem Muster-Generator 1 wird ein optisches Muster 51 erzeugt, welches in den linken und/oder den rechten Strahlengang 31, 32 einer optischen Betrachtungseinrichtung, beispielsweise eines Stereo-Operations-Mikroskops, mittels Strahlenteilern 6, 7 eingespiegelt wird. Das Muster besteht mindestens aus zwei Punkten, idealerweise aber aus zweidimensionalen Strich- oder Gittermustern. Das Muster wird über das Hauptobjektiv 10 auf das Objekt 12 projiziert. Die Reflexion beziehungsweise die Streuung des Musters am Objekt 12 wird wiederum über die Hauptoptik 10 und die Strahlenteiler 6, 7, eine Optik 4 und eine Kamera 3, beispielsweise ein CCD, detektiert (Fig.1).

Bei genauer Fokussierung des Musters auf die Fokus-Ebene 13 erscheint das Muster 51 als ein einziger Punkt bzw. eine einzige Linie bzw. ein Muster (Fig.3-5). Liegt eine Defokussierung vor, erscheint das Muster 51a, 51b doppelt in Form von zwei konjugierten Punkte bzw. konjugierten Linien bzw. konjugierten Mustern (Fig.3-5).

Die Erkennung der konjugierten Muster 51 a, 51b erfolgt über eine zeitliche und/oder räumliche und/oder geometrische und/oder spektrale Kodierung des Musters für den linken und rechten Strahlengang (31 und 32). Auf Grund der Kenntnis des Strahlengangs ist es möglich, aus den beiden Mustern den Abstand z eines oder mehrerer Objektpunkte zum Hauptobjektiv zu berechnen. In Kenntnis der Abstände ist ein direktes Fokussieren auf jeden beliebigen Objektpunkt f = z(x,y) möglich. Eine zeitliche Kodierung kann beispielsweise durch Kodierung des Musters 51 mittels einer rotierenden Blende 11 erfolgen. Die Trennung der konjugierten Signale erfolgt durch Subtraktion des normalen Bildsignals vom Gesamtsignal jeweils mit dem Muster 51a, 51b des rechten und des linken Strahlenenganges. In einer Systemeinheit 61 wird mit geeigneten Algorithmen die Relation konjugierter Musterelemente bestimmt und rechnerisch der Abstand Hauptobjektiv - Objekt, z1 bis z5 (Fig.7, Fig.8) punktweise ermittelt.

Ist das erzeugte und abgebildete Muster 51 zweidimensional ausgebildet, so ist die Oberflächenstruktur des Objektes 12 rechnerisch ermittelbar. Dies erlaubt, die Fokus-Ebene 13 hinsichtlich der Struktur der Objektoberfläche 12 oder einzelner Objekt-Punkte zu fokussieren.

Die Fokussierung auf die definierte Fokus-Ebene 13 erfolgt mittels einer hinlänglich bekannten Fokussiereinrichtung.

Die Auswahl des zu definierenden Objektpunktes kann elektronisch, - beispielsweise durch Blickrichtung der Augen, durch Analyse der Bewegungen eines Instrumentes im Bildfeld, durch Joystick, Mauszeiger oder dergleichen erfolgen - oder manuell vordefiniert werden.

Eine besondere Weiterentwicklung der Erfindung besteht darin, dass der Kamera 3 eine eigens fokussierbare Optik vorgelagert wird, um Fokusänderungen des Messsystems nicht über das Hauptobjektiv vornehmen zu müssen. Dies ermöglicht eine vom Hauptstrahlengang unabhängige Fokussierung für einen zweiten Betrachter (Fig.6) und/oder eine vom Hauptstrahlengang unabhängige Fokus-Bestimmung. In diesem Fall werden die Fokusparameter beider Optiken bei der Berechnung der Objektabstände berücksichtigt.

Insgesamt ist das Messsystem aufgrund der Fähigkeit, ein eigenes Lichtmuster zu erzeugen, praktisch unabhängig von den Objekteigenschaften einsetzbar.

### Bezugszeichenliste

- 1: Muster-Generator
- 2: Optik für (1)
- 3: Kamera (z.B. CCD)
- 4: Optik für (3)
- 5: Strahlenteiler (1)
- 6: Strahlenteiler rechter Strahlengang
- 7: Strahlenteiler linker Strahlengang
- 8: Zoom-Optik linker Strahlengang
- 9: Zoom-Optik rechter Strahlengang
- 10: Hauptobjektiv
- 11: Blende (rotierender Shutter)
- 12: Objekt-Oberfläche
- 13: Fokus-Ebene (Objekt)
- 13 a, b: positiv bzw. negativ defokussierte Ebenen
- 21: optische Achse von (10)
- 22: optische Achse linker Strahlengang
- 23: optische Achse rechter Strahlengang
- 24: optische Achse von (1)
- 25: optische Achse von (3)
- 30: Strahlengang von (1)
- 31: linker Strahlengang
- 32: rechter Strahlengang
- 33: Strahlengang Kamera / CCD
- 51: fokussiertes Muster
- 51 a, b: konjugierte Musterelemente (defokussierte Muster)
- 52 a, b: eingespiegeltes punktförmiges Muster
- 61: Bildverarbeitungseinheit
- 62: Fokussiereinrichtung für (10)
- 63: Fokussiereinrichtung für (3)
- Z1-Z5: Abstände zwischen (10) und (12)

## Patentansprüche

1. Verfahren zur Bestimmung eines oder mehrerer Z-Abstände von einer Objekt-Oberfläche (12) zu einer Bezugsebene, beispielsweise der Hauptebene des Hauptobjektivs (1) eines Mikroskops, **dadurch gekennzeichnet, dass** zumindest zwei Strahlen und/oder mehrstrahlige oder gitterförmige Muster (51) mittels eines Muster-Generators (1) erzeugt und auf das Objekt (12) projiziert werden und dass die Abbildungen der Strahlen bzw. Muster mittels einer Kamera (3) - beispielsweise ein CCD und eine Bildverarbeitungseinheit (61) - detektiert und sodann auf Grund des Abstandes zwischen den jeweils konjugierten Musterelementen (51a, 51 b) rechnerisch der z-Abstand bzw. die z-Abstände vom Hauptobjektiv (10) zum Objekt bzw. zur Fokus-Ebene (12), beispielsweise über die gesamte Objekt-Oberfläche (12), bestimmt wird bzw. werden.

2. Verfahren nach Anspruch 1, **dadurch gekennzeichnet, dass** auf die rechnerisch ermittelte Fokus-Ebene (12) selbsttätig mittels einer Fokussiereinrichtung (62) fokussiert wird.

3. Verfahren nach Anspruch 1, **dadurch gekennzeichnet, dass** projizierte konjugierte Musterelemente (51) detektiert und rechnerisch mittels vorgegebener mathematischer Algorithmen erkannt werden.

4. Verfahren nach Anspruch 1, **dadurch gekennzeichnet, dass** über die Bestimmung der Entfernung zwischen verschiedenen projizierten konjugierten Musterelementen (51) die Fokus-Ebene (12) bestimmt wird.

5. Verfahren nach einem der vorhergehenden Ansprüche, **dadurch gekennzeichnet, dass** das oder die Muster (51) das gesamte oder einen Teil des Gesichtsfeldes des Betrachters umfasst oder umfassen.

6. Verfahren nach einem der vorhergehenden Ansprüche, **dadurch gekennzeichnet, dass** die Farbe des eingespiegelten Musters (51) im sichtbaren und/oder im für das menschliche Auge unsichtbaren Lichtwellenlängen-Bereich liegt.

7. Verfahren nach einem der vorhergehenden Ansprüche, **dadurch gekennzeichnet, dass** das eingespiegelte Muster (51) zeitlich und/oder räumlich und/oder geometrisch und/oder spektral kodiert wird.

8. Verfahren nach einem der vorhergehenden Ansprüche, **dadurch gekennzeichnet, dass** konjugierten Musterelementen (52) ein Code - vorzugsweise eine Blinkfrequenz und/oder eine Lichtwellenlänge - zugewiesen wird, welcher eine einfache Bestimmung der zueinander gehörenden konjugierten Musterelemente erlaubt.

9. Verfahren nach einem der vorhergehenden Ansprüche, **dadurch gekennzeichnet, dass** über die Kodierung das Vorzeichen der Abweichung von der Fokus-Ebene (12) ermittelt wird.

10. Verfahren nach einem der vorhergehenden Ansprüche zur Durchführung an einem Stereo-Mikroskop, **dadurch gekennzeichnet, dass** konjugierte Musterelemente abwechselnd in den linken (31) oder rechten (32) Strahlengang, beziehungsweise in den rechten (32) oder linken (31) oder gleichzeitig in beide Strahlengänge eingespiegelt werden.

11. Verfahren nach einem der vorhergehenden Ansprüche, **dadurch gekennzeichnet, dass** die zeitliche Kodierung abwechselnd zwischen den konjugierten Musterelementen erfolgt und dadurch durch Subtraktion des reinen Bildsignals ein reines Muster der einzelnen konjugierten Elemente zur Auswertung entsteht.

12. Verfahren nach einem der vorhergehenden Ansprüche, **dadurch gekennzeichnet, dass** die zeitliche Kodierung mittels eines Shutters, beispielsweise einer rotierenden Scheibe (11) mit durchlässigen und undurchlässigen Bereichen zwischen dem linken (31) und rechten (32) Strahlengang, erfolgt.

13. Verfahren nach einem der vorhergehenden Ansprüche, **dadurch gekennzeichnet, dass** die Fokussierung manuell durch Beobachtung der konjugierten Muster oder elektronisch geregelt erfolgt.

14. Verfahren nach einem der vorhergehenden Ansprüche, **dadurch gekennzeichnet, dass** die automatische Fokussierung ausschließlich oder zusätzlich im Kamera-Strahlengang (33) erfolgt, beispielsweise für ein Video-System.

15. Verfahren nach einem der vorhergehenden Ansprüche, **dadurch gekennzeichnet, dass** die Fokussierung im Hauptstrahlengang automatisch ständig oder situationsbedingt einmalig nach Befehl erfolgt.

16. Verfahren nach einem der vorhergehenden Ansprüche, **dadurch gekennzeichnet, dass** der so ermittelte Fokus-Wert eines Punktes, einer Fläche oder Struktur als Abstandsparameter an externe Systeme ausgegeben wird.

17. Mikroskop mit einer Einrichtung zur Bestimmung von Z-Abständen zwischen einer Objekt-Oberfläche (12) und dem Hauptobjektiv (10) **dadurch gekennzeichnet, dass** eine Musterprojektionseinrichtung vorgesehen ist, mit der im Messzustand durch das Hauptobjektiv (10) oder eine eigene Optik ein optisches Muster (51) auf das Objekt (12) projiziert wird, die eine Kamera (3), beispielsweise ein CCD, zur Erfassung der projizierten Muster (51) enthält, wobei die erfassten Muster einer elektronischen Bildverarbeitungseinheit (61) zugeführt werden und wobei die Bildverarbeitungseinheit (61) ein Programm zur Durchführung der jeweiligen vorgenannten Verfahrensschritte enthält.
